# EUROPÄISCHE PATENTSCHRIFT

(11) **EP 2 270 128 B1**
(45) Veröffentlichungstag und Bekanntmachung des Hinweises auf die Patenterteilung: **28.03.2012**
(21) Anmeldenummer: 10006707.3
(22) Anmeldetag: 29.06.2010
(51) Int. Cl.: C12M 1/107

(54) **Biogasanlage und Serviceeinrichtung für eine Biogasanlage**
Biogas facility and manhole for a biogas facility
Installation de biogaz et élement de regard pour une installation de biogaz

(30) Priorität: 29.06.2009 DE 102009031177
(43) Veröffentlichungstag der Anmeldung: 05.01.2011
(73) Patentinhaber: Uts Biogastechnik Gmbh, 85355 Hallbergmoos (DE)
(72) Erfinder: Bierer, Johann, 84405 Dorfen (DE); Rabener, Matthias, 59302 Oelde (DE); Czwaluk, Andreas, 49377 Vechta (DE)
(74) Vertreter: Schütte, Hartmut

(56) Entgegenhaltungen:
- EP-B1- 1 717 305
- DE-U1- 20 015 808
- DE-U1-202007 007 060

## Beschreibung

Die vorliegende Erfindung betrifft eine Biogasanlage und eine Serviceeinrichtung für eine Biogasanlage.

Im Stand der Technik sind verschiedene Biogasanlagen und Serviceeinrichtungen für Biogasanlagen bekannt geworden. Biogasanlagen weisen in der Regel einen oder mehrere Fermenterbehälter zur Vergärung der Gärsubstrate auf, wobei in der Regel ein Rührgerät vorgesehen ist, um die Gärsubstrate in dem Fermenterbehälter umzurühren und wenigstens teilweise zu homogenisieren.

Solche Rührgeräte verfügen meist über Rührflügel, mit denen die Gärsubstrate vermischt und durchgerührt werden. In Abhängigkeit von den zu vergärenden Substraten und den weiteren Umgebungsbedingungen, sowie den eingesetzten Materialien, müssen die Rührgeräte in gewissen Zeitabständen inspiziert und gegebenenfalls gewartet werden. Beispielsweise können einzelne oder alle Rührflügel ausgetauscht werden.

Zur Wartung eines Rührgeräts wird das an einer Montage- oder Haltestange oder dergleichen angeordnete Rührgerät aus dem Unteren Fermentationsbereich, in dem sich die Gärsubstrate befinden, in den oberen Gasbereich verfahren. Um einen Zugang zu dem Rührgerät zu erhalten, kann beispielsweise eine mit einem flexiblen Foliendach ausgerüstete Biogasanlage geöffnet und das Foliendach ganz entfernt werden, um einen bequemen Zugang zu dem Rührgerät zu erhalten. Nachteilig an einem solchen Verfahren ist allerdings der nicht unbeträchtliche Aufwand zur Entfernung des Foliendachs. Außerdem entweicht bei Öffnung des Foliendachs das sich oberhalb des Fermentationsbereichs im Gasbereich angesammelte Biogas.

Für Biogasanlagen mit einem Betondach ist mit der DE 199 51 959 A1 ist ein rechteckiger Wartungsschacht bekannt geworden, der in das Betondach eingesetzt wird und der eine Wartung des Rührgeräts ohne nennenswerte Beeinträchtigung der Gasproduktion ermöglicht. Bei Fermentern mit hohem Feststoffanteil ist der Wartungsschacht zweiteilig ausgebildet, wobei der obere Teil etwa 10 cm oberhalb des normalen Flüssigkeitsspiegels endet. In das obere Schachtteil wird im Wartungsfall ein unteres Schachtteil eingeführt, welches in die Fermentermasse eintaucht. Zur Abdichtung ist an dem oberen Schachtteil ein umlaufender Dichtkanal vorgesehen, in welchen im zusammengesetzten Zustand ein Dichtkragen des unteren Schachtteils eingreift. Nachteilig bei solchen zweiteiligen Wartungsschächten ist, dass für jeden Wartungsvorgang das untere Schachteil erneut eingeführt und gegenüber dem oberen Schachtteil abgedichtet werden und nach dem Wartungsvorgang wieder entnommen werden muss. Die Füllmenge des Fermenters muss außerdem in engen Grenzen gehalten werden, damit eine zuverlässige Abdichtung erzielt wird. Deshalb wird in dieser Druckschrift für Fermenter mit niedrigem Feststoffanteil ein einteiliger Wartungsschacht vorgeschlagen, der mindestens 10 cm bis unter den normalen Füllstand der Flüssigkeit hineinreicht. Zum Gasausgleich ist ein absperrbares Verbindungsrohr zum Gasraum des Fermenters vorgesehen. Hier ist der Aufwand im Wartungsfalle geringer als bei einem zweiteiligen Wartungsschacht. Nachteilig ist aber, dass ein solcher Wartungsschacht nur für niedrige Feststoffanteile geeignet ist. Außerdem kann es zu Ablagerungen an dem eintauchenden Schacht kommen. Nachteilig ist weiterhin, dass der Rührwiderstand und somit der Energieverbrauch dauerhaft erhöht werden.

Mit der EP 1 717 305 B1 ist ein Biogasanlagen-Serviceschacht für einen Fermenter einer Biogasanlage mit einem Foliendach bekannt geworden, wobei der Serviceschacht als gasdichte Abdeckung domartig über einer Serviceöffnung in der Fermenterbehälterabdeckung vorgesehen ist. Die in dem Fermenterbehälter vertikal gehaltene Rühreinheit kann aus dem unteren Fermentationsbereich nach oben hin in den Serviceschachtinnenraum bewegt werden. In dem Inneren des domartig ausgebildeten Serviceschachts kann nach Öffnung einer Serviceklappe an dem Serviceschacht das Rührgerät gewartet werden und es können bei Bedarf einzelne oder alle Rührflügel ausgetauscht werden.

Ein Biogasanlagen-Serviceschacht gemäß der EP 1 717 305 B1 funktioniert zuverlässig und erlaubt bei geringem Serviceaufwand die Wartung des Rührgeräts, wobei zur Wartung nicht das gesamte Foliendach entfernt werden muss. Allerdings entweicht nach dem Öffnen der Serviceklappe das sich unter Überdruck befindliche Biogas, welches sich im oberen Gasbereich des Fermenterbehälters angesammelt hat, während gleichzeitig mit der eintretenden Luft auch eine erhebliche Menge an Sauerstoff in den Fermenterbehälter gelangt, der bei Inbetriebnahme erst wieder entfernt werden muss.

Es ist vor dem beschriebenen Stand der Technik deshalb die Aufgabe der vorliegenden Erfindung, eine Serviceeinrichtung und eine Biogasanlage mit einer Serviceeinrichtung zur Verfügung zu stellen, bei welcher ein einfacher Betrieb und eine einfache Wartung durchführbar sind, während nur ein geringer Verlust an Biogas bei einer durchzuführenden Wartung entsteht.

Diese Aufgabe wird gelöst durch eine Serviceeinrichtung mit den Merkmalen des Anspruchs 1. Die erfindungsgemäße Biogasanlage ist Gegenstand des Anspruchs 10. Bevorzugte Weiterbildungen der Erfindung sind Gegenstand der jeweiligen Unteransprüche. Weitere Vorteile und Merkmale der Erfindung ergeben sich aus dem Ausführungsbeispiel.

Die erfindungsgemäße Serviceeinrichtung umfasst eine Serviceeinheit und einen Servicebereich und ist dafür vorgesehen, an einer Biogasanlage verwendet zu werden, wobei die Biogasanlage wenigstens einen Fermenterbehälter umfasst, wobei an einem Innenraum des Fermenterbehälters wenigstens ein Fermentationsbereich und wenigstens ein Gasbereich vorgesehen sind. Der Fermenterbehälter wird von wenigstens einer an dem Fermenterbehälter angeordneten Gasabschlusswandung im Wesentlichen gasdicht abgeschlossen. An der Serviceeinheit ist wenigstens eine bewegliche Dichtungseinrichtung vorgesehen, welche im bestimmungsgemäßen Einbau wenigstens zwischen einer Ruhestellung und einer Dichtungsstellung bewegbar ist. Die Dichtungseinrichtung umfasst wenigstens eine Dichtungswand aus einem flexiblen Material. Dabei ist die Dichtungseinrichtung geeignet, den Servicebereich von dem Gasbereich in der Dichtungsstellung im Wesentlichen gasdicht zu trennen, wenigstens, wenn sich die Biogasanlage im bestimmungsgemäßen Gebrauch befindet.

Die erfindungsgemäße Serviceeinrichtung hat viele Vorteile. Ein erheblicher Vorteil der erfindungsgemäßen Serviceeinrichtung ist, dass der Servicebereich in der Dichtungsstellung von dem Gasbereich gasdicht durch die flexible Dichtungswand getrennt wird. Die flexible Dichtungswand ermöglicht die Wartung eines Rührgerätes bei einem variablen Füllstand der Fermentermasse. Die Wartung kann einfach durchgeführt werden. Die flexible Länge der Dichtungswand erlaubt eine flexible Anpassung an die jeweils vorliegenden Bedingungen. Gleichzeitig ist die Dichtungseinrichtung auch in der Ruhestellung an der Serviceeinrichtung vorgesehen und muss nicht nach jeder Wartung entfernt werden. In der Ruhestellung ist die Dichtungseinrichtung typischerweise überwiegend oberhalb des Flüssigkeitsspiegels vorgesehen und taucht wenigstens nicht mehr wesentlich in die Flüssigkeit ein. Ein stark erhöhter Strömungswiderstand wird so vermieden. Auch ein Umbau und Einbau von Komponenten ist nicht für jede Wartung erforderlich.

Durch die Erfindung wird es unaufwändig und einfach ermöglicht, ein Rührgerät oder eine Rühreinrichtung zu warten, das bzw. die sich in dem Servicebereich befindet, während gleichzeitig sich das in dem Gasbereich des Fermenterbehälters befindende Biogas nicht durch den Servicebereich der Serviceeinheit nach außen entweichen kann. Dadurch wird eine unkomplizierte Wartung mit sehr geringen Gasverlusten ermöglicht. Außerdem kann bei der Wartung kein - oder es können doch nur geringe Mengen an - Sauerstoff von außen eintreten, der bei der Wiederinbetriebnahme wieder entfernt werden muss, um den richtigen Methangehalt zu gewährleisten.

Außerdem kann der Spülungszeitraum, in dem dem Servicebereich Frischluft zugeführt wird, um explosive Gasgemische zu vermeiden, sehr kurz gehalten werden. Weiterhin kann nach vollzogener Wartung der Betrieb der Biogasanlage unverzüglich ohne Ausbau von Komponenten fortgesetzt werden, wodurch insgesamt erheblich geringere Kosten und Verluste entstehen.

In einer bevorzugten Weiterbildung der Erfindung ist der Servicebereich mit dem Gasbereich wenigstens in der Ruhestellung der Dichtungseinrichtung gasdurchlässig verbunden. Das bedeutet, dass während des normalen Betriebs der Biogasanlage der Servicebereich wenigstens teilweise und insbesondere vollständig mit dem Gasbereich verbunden ist, sodass sich auch in dem Servicebereich durch die Gärsubstrate produziertes Biogas ansammeln kann. Die flexible Dichtungswand wird für den Normalbetrieb nach oben gezogen, sodass sich im Betrieb kein gesonderter Gasraum an der Serviceeinheit ergibt.

Vorzugsweise ist die Dichtungseinrichtung an der Serviceeinheit befestigt. In einer vorteilhaften Ausgestaltung ist die Serviceeinheit mit wenigstens einem Anschlussrahmen versehen, wobei die Dichtungseinrichtung an dem Anschlussrahmen befestigt ist. Insbesondere ist.der Anschlussrahmen dafür vorgesehen, an der Gasabschlusswandung oder dem Behälterdach der Biogasanlage dichtend angeschlossen zu werden.

Insbesondere ist der Anschlussrahmen an einer Tragkonsole der Serviceeinheit befestigt. Die Serviceeinheit kann beispielsweise als Serviceschacht ausgeführt sein oder einen solchen umfassen. Eine Serviceplattform kann vorgesehen sein, wobei die Serviceplattform und die Serviceeinheit insbesondere von der Tragkonsole getragen werden. Die Tragkonsole kann an der Behälterwand des Fermenterbehälters befestigt werden. Möglich ist eine Befestigung der Tragkonsole an der Behälterinnenwand oder auch an der Behälteraußenwand. Es ist ebenso möglich, die Tragkonsole mit einem separaten Fundament außerhalb des Fermenterbehälters zu stützen.

Insbesondere ist die Dichtungseinrichtung im Dichtungszustand etwa kanalartig geformt. Besonders bevorzugt besteht die Dichtungswand wenigstens teilweise aus einem gasdichten Gewebe, einem Folienschlauch oder dergleichen.

In allen Ausgestaltungen ist es besonders bevorzugt, dass die Dichtungseinrichtung sich in der Dichtungsstellung länger als in der Ruhestellung erstreckt. Vorzugsweise ist die Dichtungseinrichtung in der Dichtungsstellung wenigstens 50 % länger und insbesondere mehr als doppelt so lang wie in der Ruhestellung. Das bedeutet, dass sich der Servicebereich, der vorzugsweise wenigstens teilweise innerhalb der Dichtungseinrichtung vorgesehen ist, sich in der Dichtungsstellung erheblich länger erstreckt als in der Ruhestellung.

Es ist bevorzugt, dass die Dichtungseinrichtung einen Faltenbalg umfasst.

Die Dichtungseinrichtung kann teleskopartig verlängerbar sein und beispielsweise leicht kegelförmig vorgesehene flexible oder flexibel gasdicht miteinander verbundene Zylindersegmente aufweisen, die auseinander gefahren werden. Dazu muss der Dichtungskörper nicht zylindrisch geformt sein, sondern kann auch einen recht- oder mehreckigen oder sonst wie geformten Querschnitt umfassen. Wichtig ist, dass die Dichtungswand an der Dichtungseinrichtung im Wesentlichen gasdicht ausgebildet ist, wenn sich die Dichtungseinrichtung in der Dichtungsposition befindet.

Vorzugsweise ist wenigstens ein verstellbares Führungselement vorgesehen, welches beispielsweise als Führungsseil oder Führungskette ausgeführt sein kann. Mit dem Führungselement ist die Dichtungseinrichtung vorzugsweise verstellbar, wobei die Dichtungswand der Dichtungseinrichtung mit dem Führungselement wenigstens von der Ruhestellung in die Dichtungsstellung absenkbar ist.

Ein flexibles Führungselement und eine flexible Dichtungswand bieten erhebliche Vorteile, da sich durch das Einziehen des Führungselements die Führungswand zusammenzieht, sodass sie in der Ruhestellung nur wenig Platz einnimmt.

Vorzugsweise ist wenigstens ein Spannrahmen oder eine Spanneinheit an der Dichtungseinrichtung vorgesehen, mit der die Dichtungswand aufgespannt wird. Eine solche Spanneinheit oder ein solcher Spannrahmen erlaubt es, dass der Querschnitt der Dichtungseinrichtung im Wesentlichen unverändert bleibt, unabhängig davon, ob sich die Serviceeinrichtung mit der Dichtungseinrichtung in der Ruhestellung oder in der Dichtungsstellung befindet.

An dieser Stelle sei darauf hingewiesen, dass unter dem Begriff "Ruhestellung" der Dichtungseinrichtung eine Position verstanden wird, in der die Dichtungseinrichtung nicht aktiv ist, während die Biogasanlage selbst selbstverständlich in Betrieb sein kann und normalerweise auch ist. Die Dichtungseinrichtung wird hingegen in die Dichtungsstellung verfahren, wenn an der Serviceeinrichtung bzw. beispielsweise an einer Rühreinrichtung in dem Servicebereich der Serviceeinrichtung eine Wartung vorgenommen werden soll.

Vorzugsweise sind mehrere Spanneinheiten oder Spannrahmen vorgesehen, die in vordefinierten Abständen oder an zueinander beabstandeten Stellen die Dichtungswand der Dichtungseinrichtung aufspannen. Die einzelnen Spanneinheiten oder Spannrahmen können miteinander verbunden sein, können aber auch einzeln an der Dichtungswand vorgesehen sein. Möglich ist es auch, dass eine Spanneinheit einen spiralförmig verlaufenden Spanndraht oder dergleichen aufweist, mit welcher die Dichtungseinrichtung aufgespannt wird, wobei die Spanneinheit für einen etwa gleichbleibenden Querschnitt sorgt, während die Dichtungswand längs verschieblich ist. Der Querschnitt ist insbesondere so bemessen, dass das Rührgerät hindurchpasst.

In vorteilhaften Weiterbildungen steht wenigstens eine Gewichtseinrichtung mit dem unteren Ende der Dichtungseinrichtung in Verbindung. Dadurch wird es ermöglicht, dass bei Entlastung des Führungselements die Dichtungseinrichtung von der Ruhestellung in die Dichtungsstellung überführt wird.

Wenn das flexible Führungselement beispielsweise als Seil oder dergleichen ausgeführt ist und in der Ruhestellung wenigstens zum Teil aufgewickelt vorliegt, so kann durch ein Abwickeln des Führungselements das untere Ende der Dichtungseinrichtung automatisch nach unten verfahren werden, wenn ein entsprechendes Gewicht an dem unteren Ende der Dichtungseinrichtung vorhanden ist. Damit erfolgt die Überführung der Dichtungseinrichtung von der Ruhestellung in die Dichtungsstellung automatisch, wenn das flexible Führungselement abgelassen wird. Eine Überführung der Dichtungseinrichtung von der Dichtungsstellung in die Ruhestellung kann dadurch erfolgen, dass das flexible Führungselement eingezogen oder eingewickelt wird.

Vorzugsweise ist ein Kurbeltrieb mit dem Führungselement verbunden, um das flexible Führungselement abzulassen oder einzuholen.

Vorzugsweise ist die Dichtungseinrichtung in der Ruhestellung an der Serviceeinrichtung angeordnet.

Vorzugsweise ist das flexible Führungselement an Ösen oder dergleichen an der Dichtungseinrichtung geführt. Wenn das flexible Führungselement eingezogen wird, zieht es die Dichtungswand der Dichtungseinrichtung zusammen.

Die erfindungsgemäße Biogasanlage ist mit wenigstens einem Fermenterbehälter ausgerüstet und weist einen in einem Innenraum des Fermenterbehälters vorgesehenen Fermentationsbereich und einen Gasbereich auf. Der Fermenterbehälter wird von einer Gasabschlusswandung oder einem Behälterdach im Wesentlichen gasdicht abgeschlossen. Wenigstens eine Rühreinrichtung und wenigstens eine Serviceeinrichtung mit einem Servicebereich sind vorgesehen, wobei die Rühreinrichtung wenigstens von einer Betriebsposition in dem Fermentationsbereich in eine Wartungsposition in dem Servicebereich der Serviceeinrichtung überführbar ist. Wenigstens eine bewegliche Dichtungseinrichtung mit wenigstens einer Dichtungswand aus einem flexiblen Material ist vorgesehen, welche zwischen einer Ruhestellung und einer Dichtungsstellung verstellbar ist, wobei die Dichtungseinrichtung in der Dichtungsstellung den Servicebereich von dem Gasbereich im Wesentlichen gasdicht trennt.

Auch die erfindungsgemäße Biogasanlage weist viele Vorteile auf. Durch die Serviceeinrichtung mit der beweglichen Dichtungseinrichtung wird eine Biogasanlage zur Verfügung gestellt, bei der die Dichtungseinrichtung bequem zwischen einer Ruhestellung und einer Dichtungsstellung verstellbar ist. Ein aufwändiges Ein- und Ausbauen von Komponenten ist zur Durchführung einer Wartung nicht erforderlich. Die Überführung zwischen der Ruhestellung und der Dichtungsstellung kann unabhängig von dem Füllstand in dem Fermenter erfolgen. Dadurch kann der Servicebereich der Serviceeinrichtung gasdicht von dem Gasbereich der Biogasanlage getrennt werden, sodass nicht das gesamte im Gasbereich des Fermenterbehälters angesammelte Biogas abgelassen werden muss, um eine Wartung oder Reparatur an beispielsweise einem im Servicebereich vorhandenen Rührgerät durchzuführen.

Insbesondere ist die Biogasanlage mit einer Serviceeinrichtung ausgerüstet, wie sie zuvor beschrieben wurde.

In einer einfachen Ausgestaltung besteht die Biogasanlage praktisch aus dem Fermenterbehälter und dem daran vorgesehenen Behälterdach sowie der Serviceeinrichtung und einer Rühreinrichtung. Das von den Gärsubstraten produzierte Biogas wird gesammelt und kann gegebenenfalls über eine entsprechende Gasleitung abgeführt werden.

Besonders bevorzugt wird die Rühreinrichtung an einer Montagestange oder Haltestange oder dergleichen höhenveränderlich gehalten. Dadurch wird es ermöglicht, die Rühreinrichtung von der Betriebsposition in eine Wartungsposition zu überführen. Dabei befindet sich die Betriebsposition typischerweise innerhalb des Fermentationsbereichs des Fermenterbehälters und die Wartungsposition oberhalb des Fermentationsbereiches. Die Wartungsposition kann innerhalb des Gasbereichs vorgesehen sein oder sogar oberhalb des Behälterdachs. Oberhalb des Behälterdachs ist die Wartungsposition beispielsweise dann vorgesehen, wenn ein Serviceschacht oder dergleichen vorgesehen ist, der sich oberhalb des Behälterdachs erstreckt, wobei das Rührgerät zur Wartung von dem unteren Fermentationsbereich in den oberen Servicebereich überführt wird, um die Wartung durchzuführen.

Dabei erstreckt sich die Haltestange dann in der Regel innerhalb des Servicebereichs aus dem Fermentationsbereich in den Gasbereich und von dort aus gegebenenfalls in das Innere des Serviceschachts und gegebenenfalls darüber hinaus.

Bei einer besonders einfachen Ausgestaltung ist die Serviceeinrichtung mit einer Serviceöffnung ausgerüstet, wobei sich die Serviceöffnung praktisch auf der Oberfläche des Behälterdachs oder der Gasabschlusswandung befinden kann oder dahin vorgesehen sein kann. Ein domartiger Aufbau eines Serviceschachts ist bei einer solchen Ausgestaltung nicht unbedingt nötig. Der Bereich, in dem die Wartung durchgeführt wird, kann dann oberhalb der Gasabschlusswandung oder des Behälterdachs im Freien vorgesehen sein. Dazu wird die Rühreinrichtung an der Montagestange oder Haltestange von dem Fermentationsbereich nach oben bis oberhalb des Behälterdachs überführt, während die Dichtungseinrichtung den Servicebereich innerhalb des Behälters zuverlässig gegenüber dem Gasbereich abdichtet.

Die Gasabschlusswandung kann durch das einwandige oder doppelwandige Behälterdach gebildet werden. Möglich ist es auch, dass die Gasabschlusswandung als Gasspeicherfolie unterhalb des eigentlichen Behälterdachs vorgesehen ist. Es ist möglich, dass das Behälterdach als Foliendach ausgebildet ist, wobei es begehbar oder nicht begehbar ausgeführt sein kann. Möglich ist es auch, dass das Behälterdach wenigstens teilweise als starres Dach und z.B. als Betondach oder Betondecke ausgeführt ist. Bei einem Betondach oder einer Betondecke wird die Gasabschlusswandung in der Regel durch das Betondach oder die Betondecke selbst gebildet.

In allen Ausgestaltungen weist die Serviceeinrichtung vorzugsweise eine Tragkonsole auf, mit welcher die Serviceeinrichtung insbesondere an der inneren oder äußeren Behälterwand abgestützt wird. Auch ein gegebenenfalls vorhandener Serviceschacht wird vorzugsweise von der Tragkonsole gehalten. Vorzugsweise wird die Dichtungseinrichtung in der Ruhestellung an der Serviceeinrichtung angeordnet und dort gehalten. In der Dichtungsstellung erstreckt sich die Dichtungseinrichtung vorzugsweise von der Serviceeinrichtung aus nach unten wenigstens bis zu dem Fermentationsbereich. Dabei kann sich die Dichtungseinrichtung auch bis unterhalb der Oberfläche des Fermentationsbereiches erstrecken. Möglich ist auch, dass die Dichtungseinrichtung auf dem Fermentationsbereich aufliegt und mit dem Rand ein wenig in den Fermentationsbereich eintaucht, um eine möglichst dichte Verbindung der Dichtungseinrichtung zu dem Fermentationsbereich zu ermöglichen. Beispielsweise kann der Rand zwischen etwa 2 cm und 5 cm in die Fermentermasse eintauchen.

In allen Ausgestaltungen ist es bevorzugt, dass die Dichtungseinrichtung die Haltestange oder die Montagestange wenigstens in der Dichtungsstellung wenigstens teilweise umgibt. Dadurch wird gewährleistet, dass die Rühreinrichtung bei einer Höhenverstellung sich innerhalb der Dichtungseinrichtung nach oben bzw. nach unten bewegt und die Dichtungswand nicht durchstoßen muss, sodass einer zusätzlichen Dichtung an der Montage- oder Haltestange eingespart wird.

Wie zuvor beschrieben, kann eine Spanneinheit oder ein Spannrahmen oder mehrere solcher Spanneinheiten oder Spannrahmen vorgesehen sein, um die Dichtungseinrichtung in der Dichtungsstellung aufzuspannen.

Insgesamt bildet die Dichtungseinrichtung einen Gasvorhang, der den Bereich des Biogases in dem Fermentationsbehälter von dem Servicebereich trennt. Die eigentliche Wartung des Rührgeräts bzw. einer Rühreinrichtung kann innerhalb des Behälters erfolgen, kann aber auf dem Dach oder oberhalb des Dachs des Fermenterbehälterdachs durchgeführt werden.

In allen Fällen ist im Normalbetrieb der Biogasanlage die Dichtungseinrichtung mit der Dichtungswand hochgezogen. Um eine Wartung des Rührgerätes durchzuführen, kann das Rührgerät zur Außenwand verschwenkt werden und über die Höhenverstellung beispielsweise bis zum Anschlag hochgedreht werden. Anschlieβend oder gleichzeitig kann die als Gasvorhang dienende Dichtungswand der Dichtungseinrichtung mit Hilfe der Führungselemente der vorhandenen oder zusätzlichen Gewichte an der Dichtungswand abgesenkt werden, bis es das Gärsubstrat erreicht und gegebenenfalls leicht oder sogar mehr darin eintaucht.

Im Anschluss daran kann der Wartungsdeckel bzw. die Serviceöffnung der Serviceeinrichtung geöffnet werden und das sich im Servicebereich befindliche Restgas abgelassen werden. Danach kann das Rührgerät ganz hochgedreht werden, bis es beispielsweise oberhalb des Behälterdachs angeordnet ist und dort gewartet wird. Gleichzeitig wird das in dem Fermenterbehälter sich in dem Gasbereich befindende Biogas von der als Gasfolie dienenden Dichtungswand zurückgehalten, sodass fast kein Gasverlust entsteht und gleichzeitig eine angenehmere Wartungsarbeit ermöglicht wird. Nach der Wartung kann das Rührgerät wieder abgesenkt, die Serviceöffnung verschlossen und die Dichtungsfolie bzw. Gasfolie wieder hochgezogen und gesichert werden. Im Anschluss daran kann die Anlage wieder normal in Betrieb gehen.

Zur Versteifung der Dichtungswand der Dichtungseinrichtung kann am unteren Ende bzw. in bestimmten Abständen ein Rohr oder eine Trag- oder Spanneinheit vorgesehen sein, die die Dichtungswand versteift und aufspannt. Zum automatischen oder manuellen Hochziehen der Dichtungswand sind an den Ecken der Serviceöffnung der Serviceeinrichtung verschließbare Rohre oder dergleichen montiert, durch welche das flexible Führungselement durchgeführt sein kann.

Weitere Vorteile und Merkmale der vorliegenden Erfindung ergeben sich aus dem Ausführungsbeispiel, das mit Bezug auf die beiliegenden Figuren im Folgenden beschrieben wird:

Darin zeigen:
- Fig. 1: eine schematische Seitenansicht einer erfindungsgemä- β en Biogasanlage mit einem teilgeschnittenen Fermen- terbehälter;
- Fig. 2: eine vergrößerte Darstellung der Serviceeinrichtung der Biogasanlage nach Fig. 1;
- Fig. 3: eine schematische Darstellung der Dichtungseinrich- tung der Serviceeinrichtung nach Fig. 2;
- Fig. 4: eine schematisch vergrößerte Ansicht der Serviceein- richtung in der Ruhestellung; und
- Fig. 5: eine Darstellung der Serviceeinrichtung in der Dich- tungsstellung.

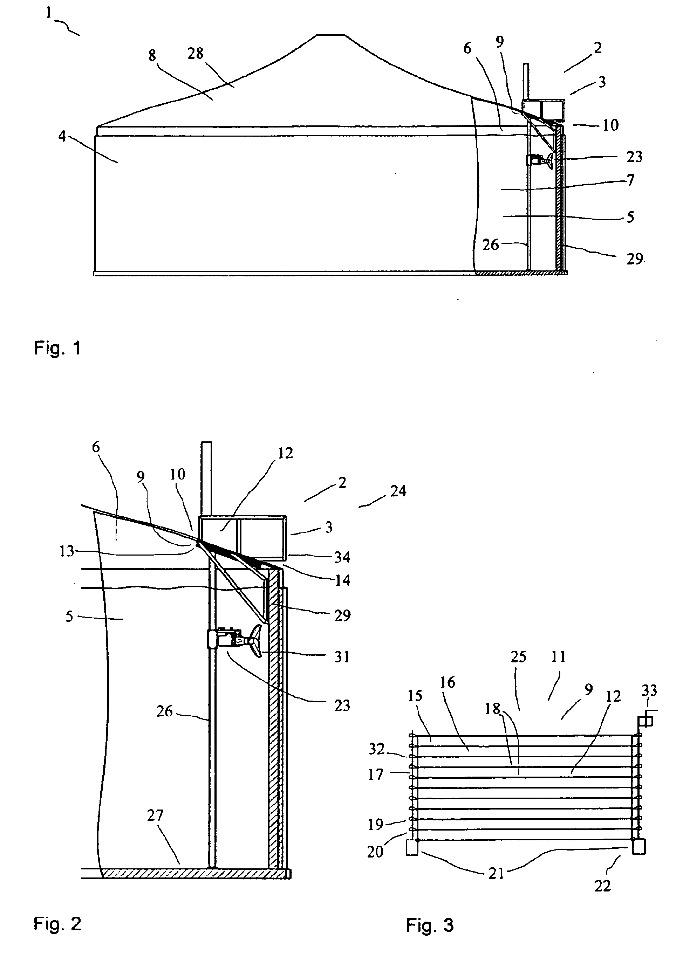

Mit Bezug auf die Figuren 1 - 5 wird im Folgenden ein Ausführungsbeispiel der Biogasanlage 1 mit einer Serviceeinrichtung 2 erläutert.

Die in Fig. 1 in einer Seitenansicht dargestellte Biogasanlage 1 umfasst einen Fermenterbehälter 4, in dem sich in dem unteren Fermentationsbereich 5 ein gärfähiges Substrat zur Erzeugung des Biogases befindet, wobei sich das entstandene Biogas in dem Gasbereich 6 oberhalb des Fermentationsbereichs 5 sammelt.

Der Fermenterbehälter 4 weist eine Behälterwand 29 und ein Behälterdach 8 auf, welches im vorliegenden Fall als flexibles Foliendach 28 ausgeführt ist. Das Foliendach 28 bildet hier die Gasabschlusswandung 8.

Um in dem Innenraum 7 des Fermenterbehälters 4 in dem Fermentationsbereich 5 das Gärsubstrat umzurühren und um den Gärprozess zu fördern, ist wenigstens ein Rührgerät als Rühreinrichtung 23 vorgesehen, welches bzw. welche an einer Haltestange 26 angeordnet ist, die sich vom Boden 27 des Fermenterbehälters 4 aus bis oberhalb des Behälterdachs 28 erstreckt.

An dem Fermenterbehälter 4 ist wenigstens eine Serviceeinrichtung 2 vorgesehen, die als Serviceeinheit oder Serviceschacht 3 ausgeführt ist.

Der hier im Ausführungsbeispiel dargestellte Serviceschacht 3 wird durch eine Tragkonsole 14 an der Innenseite der Behälterwand 29 getragen.

Der Serviceschacht 3 weist eine Plattform 34 auf, auf der eine Person zur Durchführung der Wartung sich aufhalten kann. Die Serviceeinheit 2 ist in gasdichter Weise mit dem Foliendach 28 bzw. der Gasabschlusswandung 8 verbunden.

In der in Fig. 1 und 2 dargestellten Stellung befindet sich die Dichtungseinrichtung 9 in der Ruhestellung 10, in der die Dichtungswand 15 der Dichtungseinrichtung 9 an dem Anschlussrahmen 13 der Serviceeinrichtung 2 zusammengezogen angeordnet ist. In der Ruhestellung 10 nimmt die Dichtungseinrichtung 9 nur wenig Platz ein. In dieser Stellung wird der normale Betrieb der Biogasanlage 1 durchgeführt, wobei die Rühreinrichtung 23 das Gärsubstrat in dem Fermentationsbereich 5 umrührt, während sich oberhalb des Fermentationsbereichs 5 im Gasbereich 6 das entstehende Biogas sammelt.

Die Dichtungseinrichtung 9 umfasst hier eine Dichtungswand 15, die insgesamt etwa als Faltenbalg 16 ausgeführt ist und die von der in Fig. 2 dargestellten Stellung in die in Fig. 3 dargestellte ausgefahrene Stellung überführbar ist. In Fig. 3 ist die Dichtungseinrichtung 9 in der Dichtungsstellung 11 abgebildet. Das wird hier erreicht, in dem die als Seile ausgeführten flexiblen Führungselemente 17 über den Kurbeltrieb 33 abgelassen werden, wobei das Eigengewicht der Dichtungseinrichtung 9 oder aber auch zusätzliche Gewichte 21 dafür sorgen, dass die Dichtungswand 15 abgesenkt wird. Die Dichtungswand 15 kann über zusätzliche Spanneinheiten 18 aufgespannt werden, die sich in bestimmten Abständen an der Dichtungswand befinden. Über Befestigungsösen 32 wird die Dichtungswand 15 mit dem als Seil ausgeführten Führungselement 17 verbunden.

Wird das Führungselement 17 abgelassen, so bewirkt das Eigengewicht oder die zusätzlichen Gewichte 21, dass sich die Dichtungswand 15 absenkt, bis die Dichtungswand 15 die Oberfläche des Fermentationsbereichs 5 erreicht und gegebenenfalls leicht darin eintaucht. Es wird ein dichter Abschluss des sich in dem Inneren der Dichtungseinrichtung befindenden Servicebereichs 12 von dem Gasbereich 6 erzielt.

In dieser Dichtungsstellung 11 kann eine Wartung durchgeführt werden. Dazu wird das Rührgerät 23 nach außen zur Behälterwand verfahren, dort verschwenkt und anschließend hochgefahren, bis es sich oberhalb des Fermentationsbereichs in dem Servicebereich 12 befindet, der durch die abgesenkte Dichtungswand 15 gasdicht von dem Gasbereich 6 abgetrennt ist.

In dem dargestellten Ausführungsbeispiel wird das Rührgerät 23 bis oberhalb des Behälterdachs 28 verfahren, bis es sich innerhalb des Teils des Servicebereichs 12 befindet, der innerhalb des Serviceschachts 3 vorgesehen ist. Dort kann anschließend die Serviceöffnung geöffnet werden und nach dem Ablassen des im Servicebereich noch vorhandenen Biogases kann das Rührgerät bequem zugänglich gewartet werden.

Die Dichtungseinrichtung kann an einer Seite gelenkig gelagert sein, während die andere Seite verschwenkbar ist, um die Dichtungswand zwischen der Ruhestellung 10 und der Dichtungsstellung 11 zu verfahren. Es ist möglich, dass der Schwenkpunkt leicht in der Höhe der maximalen Füllstandshöhe oder leicht darunter angeordnet ist, wobei sich der wesentliche Teil und insbesondere wenigstens die Hälfte, dreiviertel oder mehr der Dichtungseinrichtung 9 in der Ruhestellung 10 oberhalb der Fermentermasse befindet.

In einer alternativen Ausgestaltung erstreckt sich der Serviceschacht beispielsweise nicht domartig oberhalb des Behälterdachs, sondern es wird in einem einfachen Falle eine Serviceöffnung in dem Behälterdach vorgesehen, die nach dem Öffnen ein Hochfahren des Rührgeräts bis oberhalb des Behälterdachs erlaubt, woraufhin das Rührgerät im Freien oberhalb des Behälterdachs 28 gewartet werden kann.

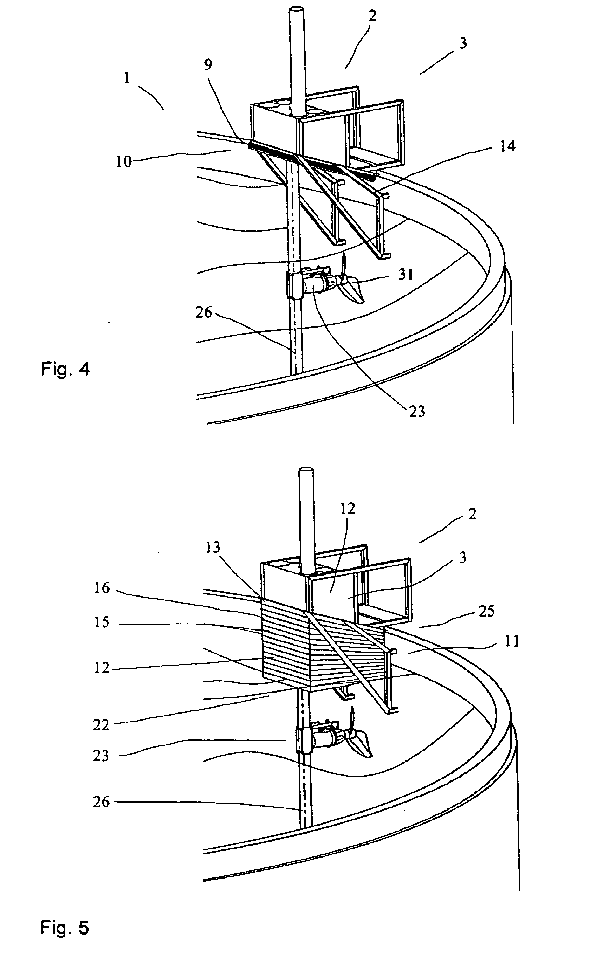

In Fig. 4 ist nochmals die Betriebsposition dargestellt, in der sich die Dichtungseinrichtung in der Ruhestellung 10 befindet und die Biogasanlage sich in einem normalen Betrieb befinden kann. Zur besseren Übersichtlichkeit wurde das Foliendach transparent dargestellt, um in das Innere des Fermenterbehälters 4 Einblick zu erhalten.

In Fig. 5 ist in ähnlicher Weise die Dichtungsstellung 11 dargestellt, in der die Wartung 25 durchgeführt werden kann. Dabei ist die als Gasvorhang dienende Dichtungswand 15 der Dichtungseinrichtung 9 bis auf die Höhe des Fermentationsbereichs 5 abgesenkt worden.

Insgesamt erlaubt die Erfindung eine besonders komfortable Wartung eines Rührgeräts und weiterer Komponenten. Dabei entsteht nur ein sehr geringer Gasverlust durch die Durchführung der Wartung, wobei nur wenig Sauerstoff in die Anlage gelangt. Der Einsatz der Erfindung ist auch wirtschaftlich interessant.

### Bezugszeichenliste:

- 1: Biogasanlage
- 2: Serviceeinrichtung
- 3: Serviceeinheit, Serviceschacht
- 4: Fermenterbehälter
- 5: Fermentationsbereich
- 6: Gasbereich
- 7: Innenraum
- 8: Gasabschlusswandung, Behälterdach
- 9: Dichtungseinrichtung
- 10: Ruhestellung
- 11: Dichtungsstellung
- 12: Servicebereich
- 13: Anschlussrahmen
- 14: Tragkonsole
- 15: Dichtungswand
- 16: Faltenbalg
- 17: Führungselement
- 18: Spannrahmen
- 19: Stelle
- 20: Stelle
- 21: Gewichtseinrichtung
- 22: Ende
- 23: Rühreinrichtung
- 24: Betriebsposition
- 25: Wartungsposition
- 26: Haltestange
- 27: Boden
- 28: Foliendach
- 29: Behälterwand
- 30: Verstelleinrichtung
- 31: Rührflügel
- 32: Befestigungsöse
- 33: Kurbeltrieb
- 34: Plattform

## Patentansprüche

1. Serviceeinrichtung (2) mit einer Serviceeinheit (3) und einem Servicebereich (12), welche dafür vorgesehen ist, an einer Biogasanlage (1) verwendet zu werden, wobei die Biogasanlage (1) wenigstens einen Fermenterbehälter (4) umfasst und einen Fermentationsbereich (5) und einen Gasbereich (6) an einem Innenraum (7) des Fermenterbehälters (4) aufweist, wobei der Fermenterbehälter (4) von wenigstens einer an dem Fermenterbehälter (4) angeordneten Gasabschlusswandung (8) im Wesentlichen gasdicht abgeschlossen wird,
wobei wenigstens eine Dichtungseinrichtung (9) vorgesehen ist, welche geeignet ist, den Servicebereich (12) von dem Gasbereich (6) im Wesentlichen gasdicht zu trennen, **dadurch gekennzeichnet,**
**dass** die Dichtungseinrichtung (9) wenigstens eine Dichtungswand aus einem flexiblen Material umfasst und im bestimmungsgemäßen Einbau wenigstens zwischen einer Ruhestellung (10) und einer Dichtungsstellung (11) bewegbar ist.

2. Serviceeinrichtung (2) nach Anspruch 1, wobei der Servicebereich (12) mit dem Gasbereich (6) in der Ruhestellung (10) gasdurchlässig verbunden ist.

3. Serviceeinrichtung (2) nach Anspruch 1 oder 2, wobei die Serviceeinheit (3) einen Anschlussrahmen (13) umfasst und die Dichtungseinrichtung (9) an dem Anschlussrahmen (13) befestigt ist.

4. Serviceeinrichtung (2) nach mindestens einem der vorhergehenden Ansprüche 2 oder 3, wobei der Anschlussrahmen (13) an einer Tragkonsole (14) der Serviceeinheit (3) befestigt ist.

5. Serviceeinrichtung (2) nach mindestens einem der vorhergehenden Ansprüche, wobei die Dichtungseinrichtung (9) einen Faltenbalg (16) umfasst und wobei die Dichtungseinrichtung (9) in der Dichtungsstellung (11) insbesondere länger als in der Ruhestellung (10) ist.

6. Serviceeinrichtung (2) nach mindestens einem der vorhergehenden Ansprüche, wobei wenigstens ein verstellbares Führungselement (17), wie ein Führungsseil (17) oder eine Führungskette vorgesehen ist, mit welcher die Dichtungseinrichtung (9) verstellbar ist, wobei die Dichtungswand (15) der Dichtungseinrichtung (9) mit dem Führungselement (17) wenigstens von der Ruhestellung (10) in die Dichtungsstellung (11) absenkbar ist.

7. Serviceeinrichtung (2) nach mindestens einem der vorhergehenden Ansprüche, wobei wenigstens ein Spannrahmen (18) an der Dichtungseinrichtung (9) vorgesehen ist, der die Dichtungswand (15) aufspannt, wobei vorzugsweise mehrere Spannrahmen (18) an der Dichtungseinrichtung (9) vorgesehen sind, welche die Dichtungswand (15) an mehreren zueinander beabstandeten Stellen (19, 20) aufspannen.

8. Serviceeinrichtung (2) nach mindestens einem der vorhergehenden Ansprüche, wobei wenigstens eine Gewichtseinrichtung (21) mit dem unteren Ende (22) der Dichtungseinrichtung (9) in Verbindung steht, um bei Entlastung des Führungselements 17) die Dichtungseinrichtung (9) von der Ruhestellung (10) in die Dichtungsstellung (11) zu überführen.

9. Serviceeinrichtung (2) nach mindestens einem der vorhergehenden Ansprüche, wobei die Dichtungseinrichtung (9) in der Ruhestellung (10) an der Serviceeinrichtung (2) angeordnet ist.

10. Biogasanlage (1) mit wenigstens einem Fermenterbehälter (4) und einem in einem Innenraum (7) des Fermenterbehälters (4) vorgesehenen Fermentationsbereich (5) und einem Gasbereich (6), der von wenigstens einer an dem Fermenterbehälter (4) angeordneten Gasabschlusswandung (8) im Wesentlichen gasdicht abgeschlossen wird,
wobei wenigstens eine Rühreinrichtung (23) und wenigstens eine Serviceeinrichtung (2) mit einem Servicebereich (12) vorgesehen sind, wobei die Rühreinrichtung (23) wenigstens von einer Betriebsposition (24) in dem Fermentationsbereich (5) in eine Wartungsposition (25) in dem Servicebereich (12) der Serviceeinrichtung (2) überführbar ist,
**dadurch gekennzeichnet,**
**dass** wenigstens eine bewegliche Dichtungseinrichtung (9) vorgesehen ist, welche wenigstens eine Dichtungswand aus einem flexiblen Material umfasst und zwischen einer Ruhestellung (10) und einer Dichtungsstellung (11) verstellbar ist, wobei die Dichtungseinrichtung (9) in der Dichtungsstellung (11) den Servicebereich (12) von dem Gasbereich (6) im Wesentlichen gasdicht trennt.

11. Biogasanlage (1) nach mindestens einem der vorhergehenden Ansprüche, wobei sich die Haltestange (26) von einem Boden (27) des Fermenterbehälters bis wenigstens in den Servicebereich (12) der Serviceeinrichtung (2) erstreckt.

12. Biogasanlage (1) nach mindestens einem der vorhergehenden Ansprüche, wobei die Serviceeinrichtung (2) einen Serviceschacht (3) und eine Tragkonsole (14) umfasst und wobei die Serviceeinrichtung (2) insbesondere an der inneren oder äußeren Behälterwand (29) abgestützt wird.

13. Biogasanlage (1) nach mindestens einem der vorhergehenden Ansprüche, wobei die Dichtungseinrichtung (9) in der Ruhestellung (10) an der Serviceeinrichtung (2) angeordnet ist und wobei die Dichtungseinrichtung (9) sich in der Dichtungsstellung (11) von der Serviceeinrichtung (2) aus wenigstens bis zu dem Fermentationsbereich (5) erstreckt.

14. Biogasanlage (1) nach mindestens einem der vorhergehenden Ansprüche, wobei die Dichtungseinrichtung (9) die Haltestange (26) wenigstens in der Dichtungsstellung (11) wenigstens teilweise umgibt und/oder wobei an der Haltestange (26) eine Verstelleinrichtung (30) zur Verstellung der Rühreinrichtung (23) vorgesehen ist.

15. Biogasanlage (1) nach mindestens einem der vorhergehenden Ansprüche, wobei wenigstens ein Spannrahmen (18) an der Dichtungseinrichtung (9) vorgesehen ist, der die Dichtungswand (15) aufspannt, wobei vorzugsweise mehrere Spannrahmen (18) an der Dichtungseinrichtung (9) vorgesehen sind, welche die Dichtungswand (15) an mehreren zueinander beabstandeten Stellen aufspannen.

## Claims

1. Servicing device (2) having a servicing unit (3) and a servicing section (12) provided to be employed in a biogas plant (1), the biogas plant (1) comprising at least one fermenter tank (4) and a fermentation section (5) and a gas section (6) at an interior space (7) of the fermenter tank (4) which Fermenter tank (4) is closed substantially gas-tight by at least one gas closing wall (8) disposed at the fermenter tank (4), wherein at least one sealing device (9) is provided which is suitable to effect a substantially gas-tight separation of the servicing section (12) from the gas section (6), **characterized in that** the sealing device (9) comprises at least one retention wall of a flexible material and when installed as intended is movable at least between an inoperative position (10) and a sealing position (11).

2. The servicing device (2) according to claim 1 wherein the servicing section (12) is connected with the gas section (6) to be gas-permeable in the inoperative position (10).

3. The servicing device (2) according to claim 1 or 2 wherein the servicing unit (3) comprises an attachment frame (13) and the sealing device (9) is attached to the attachment frame (13).

4. The servicing device (2) according to at least one of the preceding claims 2 or 3 wherein the attachment frame (13) is attached to a carrying console (14) of the servicing unit (3).

5. The servicing device (2) according to at least one of the preceding claims wherein the sealing device (9) comprises bellows (16) and wherein the sealing device (9) is in particular longer in the sealing position (11) than in the inoperative position (10).

6. The servicing device (2) according to at least one of the preceding claims wherein at least one displaceable guide member (17) such as a guide rope (17) or a guide chain is provided by means of which the sealing device (9) is displaceable wherein the retention wall (15) of the sealing device (9) can be descended by means of the guide member (17) at least from the inoperative position (10) to the sealing position (11).

7. The servicing device (2) according to at least one of the preceding claims wherein at least one mounting frame (18) is provided at the sealing device (9) for spreading the retention wall (15) wherein preferably multiple mounting frames (18) are provided at the sealing device (9) which spread the retention wall (15) in multiple positions (19, 20) spaced apart from one another.

8. The servicing device (2) according to at least one of the preceding claims wherein at least one weighting device (21) is connected with the lower end (22) of the sealing device (9) to transfer the sealing device (9) from the inoperative position (10) to the sealing position (11) as the guide member (17) is relieved.

9. The servicing device (2) according to at least one of the preceding claims wherein the sealing device (9) is disposed at the servicing device (2) in the inoperative position (10).

10. Biogas plant (1) having at least one fermenter tank (4) and a fermentation section (5) provided in an interior space (7) of the fermenter tank (4) and a gas section (6) which is closed substantially gas-tight by at least one gas closing wall (8) disposed at the fermenter tank (4) wherein at least one agitator device (23) and at least one servicing device (2) with a servicing section (12) are provided wherein the agitator device (23) can be transferred at least from an operating position (24) in the fermentation section (5) to a maintenance position (25) in the servicing section (12) of the servicing device (2), **characterized in that** at least one movable sealing device (9) is provided comprising at least one retention wall of a flexible material and is displaceable between an inoperative position (10) and a sealing position (11) wherein in the sealing position (11) the sealing device (9) separates the servicing section (12) from the gas section (6) substantially gas-tight.

11. The biogas plant (1) according to at least one of the preceding claims wherein the holding rod (26) extends from a bottom (27) of the fermenter tank at least as far as the servicing section (12) of the servicing device (2).

12. The biogas plant (1) according to at least one of the preceding claims wherein the servicing device (2) comprises a servicing shaft (3) and a carrying console (14) and wherein the servicing device (2) is in particular supported on the inner or outer tank wall (29).

13. The biogas plant (1) according to at least one of the preceding claims wherein the sealing device (9) in the inoperative position (10) is disposed on the servicing device (2) and wherein the sealing device (9) in the sealing position (11) extends from the servicing device (2) at least as far as the fermentation section (5).

14. The biogas plant (1) according to at least one of the preceding claims wherein the sealing device (9) surrounds at least part of the holding rod (26) at least in the sealing position (11) and/or wherein the holding rod (26) is provided with a displacement device (30) for displacing the agitator device (23).

15. The biogas plant (1) according to at least one of the preceding claims wherein at least one mounting frame (18) is provided at the sealing device (9) for spreading the retention wall (15) wherein preferably multiple mounting frames (18) are provided at the sealing device (9) which spread the retention wall (15) in multiple positions spaced apart from one another.

## Revendications

1. Dispositif de service (2) avec une unité de service (3) et une zone de service (12), prévu pour être utilisé dans une installation de production de biogaz (1), l'installation de production de biogaz (1) comprenant au moins un réservoir de fermenteur (4) et présentant une zone de fermentation (5) ainsi qu'une zone de gaz (6) dans un espace intérieur (7) du réservoir de fermenteur (4), le réservoir de fermenteur (4) étant fermé sensiblement de façon hermétique au gaz par au moins une paroi d'arrêt de gaz (8) agencée dans le réservoir de fermenteur (4), au moins un dispositif d'étanchéité (9) étant prévu, lequel est apte à séparer sensiblement de façon hermétique au gaz la zone de service (12) de la zone de gaz (6)
**caractérisé en ce**
**que** le dispositif d'étanchéité (9) comprend au moins une paroi d'étanchéité en matériau souple, et qu'il est, en assemblage conventionnel, mobile au moins entre une position de repos (10) et une position d'étanchéité (11).

2. Dispositif de service(2) selon la revendication 1, la zone de service (12) étant reliée à la zone de gaz (6) de manière à laisser passer le gaz en position de repos (10).

3. Dispositif de service (2) selon la revendication 1 ou 2, l'unité de service (3) comprenant un cadre de raccordement (13) et le dispositif d'étanchéité (9) étant fixé au cadre de raccordement (13).

4. Dispositif de service (2) selon au moins l'une quelconque des revendications précédentes 2 ou 3, le cadre de raccordement (13) étant fixé à une console (14) de l'unité de service (3).

5. Dispositif de service (2) selon au moins l'une quelconque des revendications précédentes, le dispositif d'étanchéité (9) comprenant un soufflet plissé (16) et le dispositif d'étanchéité (9) étant, particulièrement en position d'étanchéité (11), plus long qu'en position de repos (10).

6. Dispositif de service (2) selon au moins l'une quelconque des revendications précédentes, au moins un élément de guidage(17) réglable, tel une corde de guidage (17) ou une chaîne de guidage, étant prévu et au moyen duquel le dispositif d'étanchéité (9) est réglable, la paroi d'étanchéité (15) du dispositif d'étanchéité (9) étant, à l'aide de l'élément de guidage (17) au moins abaissable de la position de repos (10) en position d'étanchéité (11).

7. Dispositif de service (2) selon au moins l'une quelconque des revendications précédentes, au moins un cadre de serrage (18) étant prévu au niveau du dispositif d'étanchéité (9) et fixant la paroi d'étanchéité (15), de préférence plusieurs cadres de serrage (18) étant prévus au niveau du dispositif d'étanchéité (9), lesquels fixent la paroi d'étanchéité (15) en plusieurs points (19, 20) espacés les uns des autres.

8. Dispositif de service (2) selon au moins l'une quelconque des revendications précédentes, au moins un dispositif de poids (21) étant relié à l'extrémité inférieure (22) du dispositif d'étanchéité (9) afin de ramener le dispositif d'étanchéité (9), lors de la décharge de l'élément de guidage (17), de la position de repos (10) en position d'étanchéité (11).

9. Dispositif de service (2) selon au moins l'une quelconque des revendications précédentes, le dispositif d'étanchéité (9) étant placé au niveau du dispositif de regard (2) en position de repos (10).

10. Installation de production de biogaz (1) avec au moins un réservoir de fermenteur (4) et une zone de fermentation (5) prévue dans un espace intérieur (7) du réservoir de fermenteur (4) et une zone de gaz (6) fermée sensiblement de façon hermétique au gaz par au moins une paroi d'arrêt de gaz (8) placée au niveau du réservoir de fermenteur (4),
au moins un dispositif mélangeur (23) et au moins un dispositif de service (2) étant prévus avec une zone de service (12), le dispositif mélangeur (23) pouvant être ramené au moins d'une position de marche (24) dans la zone de fermentation (5) à une position d'entretien (25) dans la zone de service (12) du dispositif de regard (2) **caractérisé en ce**
**qu'**au moins un dispositif d'étanchéité (9) mobile étant prévu, comprenant au moins une paroi d'étanchéité en matériau souple et réglable entre une position de repos (10) et une position d'étanchéité (11), le dispositif d'étanchéité (9) en position d'étanchéité (11) séparant sensiblement de façon hermétique la zone de service (12) de la zone de gaz (6).

11. Installation de production de biogaz (1) selon au moins l'une quelconque des revendications précédentes, le poteau de fixation (26) allant d'un fond (27) du réservoir de fermenteur (4) au moins jusqu'à la zone de service (12) du dispositif de service.

12. Installation de production de biogaz (1) selon au moins l'une quelconque des revendications précédentes, le dispositif de service (2) comprenant un élément de regard (3) et une console (14), et le dispositif de service (2) étant soutenu particulièrement à la paroi intérieure ou extérieure du réservoir (29).

13. Installation de production de biogaz (1) selon au moins l'une quelconque des revendications précédentes, le dispositif d'étanchéité (9) étant placé en position de repos (10) au niveau du dispositif de service (2) et le dispositif d'étanchéité (9) allant en position d'étanchéité (11) du dispositif de service (2) à la zone de fermentation (5) au moins.

14. Installation de production de biogaz (1) selon au moins l'une quelconque des revendications précédentes, le dispositif d'étanchéité (9) entourant au moins partiellement le poteau de fixation (26) au moins en position d'étanchéité (11) et/ou un dispositif de réglage (30) étant prévu au niveau du poteau de fixation (26) pour le réglage du dispositif mélangeur (23).

15. Installation de production de biogaz (1) selon au moins l'une quelconque des revendications précédentes, un cadre de serrage (18) étant prévu sur le dispositif d'étanchéité (9) et fixant la paroi d'étanchéité (15), de préférence plusieurs cadres de serrage (18) étant prévus au niveau du dispositif d'étanchéité (9), lesquels fixent la paroi d'étanchéité (15) en plusieurs points (19, 20) espacés les uns des autres.
